# EUROPÄISCHE PATENTANMELDUNG

(11) **EP 1 174 076 A2**
(43) Veröffentlichungstag der Anmeldung: **23.01.2002**
(21) Anmeldenummer: 01115533.0
(22) Anmeldetag: 28.06.2001
(51) Int. Cl.: A61B 1/005

(54) **Vorrichtung zur automatischen Durchführung von diagnostischen und/oder therapeutischen Aktionen in Körperhöhlungen**

(30) Priorität: 18.07.2000 DE 10035320; 18.08.2000 DE 10040366
(71) Anmelder: BIOTRONIK Mess- und Therapiegeräte GmbH & Co Ingenieurbüro Berlin, 12359 Berlin (DE)
(72) Erfinder: Schaldach, Max, Prof. Dr., Verstorben (DE); Kranz, Curt, Dr., 14163 Berlin (DE); Gobrecht, Jens, Dr., 5412 Gebenstorf (CH)
(74) Vertreter: Hübner, Gerd, Dipl.-Phys.

(57) **Zusammenfassung**

Eine Vorrichtung zur automatischen Durchführung von diagnostischen und/oder therapeutischen Aktionen in Körperhöhlungen (6) ist versehen mit einer extrakorporal anzuordnenden Basis-Steuereinheit (1) und einem damit gekoppelten, in die entsprechende Körperhöhlung einführbaren, katheterartigen Aktuator (3), der im Bereich seines distalen Endes (35) mit einer diagnostisch aktiven Detektor- und/oder therapeutisch aktiven Behandlungseinrichtung (36) versehen ist, sowie zumindest im Bereich seines distalen Endes (35) mit einer von der Basis-Steuereinheit (1) angesteuerten Antriebseinrichtung (43) zur Vorschub- und/oder Krümmungssteuerung des Aktuators (3) versehen ist.

## Beschreibung

Die Erfindung betrifft eine Vorrichtung zur automatischen Durchführung von diagnostischen und/oder therapeutischen Aktionen in Körperhöhlungen. Darunter fallen insoweit Katheter, Kanülen, Elektroden, Endoskope oder ähnliche Geräte, wobei unter Körperhöhlungen alle Arten von im menschlichen oder tierischen Körper zu untersuchende Organe und Gefäße zu verstehen sind.

Ein grundsätzliches Problem bei solchen medizinischen Vorrichtungen ist die Steuerung zu dem gewünschten Diagnose- oder Behandlungsort innerhalb der Körperhöhlung. Übliche mechanische Steuerungseinrichtungen sind beispielsweise durch steife Mandrindrähte als Seele oder durch Zugseile in einem Katheter gebildet, wobei durch einen Zug an diesen Elementen die Krümmung der Katheterspitze und damit die Vorschubrichtung veränderbar sind.

Aus dem Stand der Technik sind ferner verschiedene Gerätschaften der vorstehend erörterten Art bekannt, bei denen Piezoelemente zur Steuerung eingesetzt werden. So offenbart die US-A-6 066 094 ein Verfahren und eine Vorrichtung zur Vermessung des Herzens, bei der mit einem steuerbaren Katheter mehrere Punkte im Herzen angefahren und Messungen vorgenommen werden können. Die US-A-5 415 633 betrifft ein implantierbares Gerät, das am distalen Enden eine Art Wurmfortsatz besitzt, der mittels Piezo-Elementen steuerbar ist. Die US-A-5 419 312 beschreibt ein Endoskop, das unter anderem zur Steuerung und Lenkung am distalen Ende mit Piezokeramiken beschichtet ist. Damit wird eine aktive Biegbarkeit des Endoskops erreicht. Eine ähnliche Vorrichtung offenbart die US-A-6 048 307. Schließlich betrifft die DE-A-195 45 946 einen so bezeichneten Piezomotor, der auf der Basis eines längenveränderlichen Piezoelementes arbeitet.

Der Erfindung liegt nunmehr die Aufgabe zugrunde, eine medizintechnische Vorrichtung der eingangs erörterten Art so weiterzubilden, daß ihr Aktuator in der Lage ist, weitgehend selbsttätig das oder die Zielgebiete anzufahren und dort entsprechende diagnostische oder therapeutische Maßnahmen zu ergreifen.

Zur Lösung dieser Aufgabe ist eine vorzugsweise extrakorporal anzuordnende Basis-Steuereinheit vorgesehen, mit der ein katheterartiger, in die entsprechende Körperhöhlung einführbarer Aktuator gekoppelt ist. Letzterer ist im Bereich seines distalen Endes mit einer diagnostisch aktiven Detektoreinrichtung bzw. einer therapeutisch aktiven Behandlungseinrichtung versehen, wie beispielsweise einer Ablationselektrode, einem Dilatationsballon oder dergleichen. Ferner ist der Aktuator zumindest im Bereich seines distalen Endes und - falls dies aus Manövrierbarkeitsgründen erforderlich ist - auch gegebenenfalls bis über seine ganze Länge mit einer von der Basis-Steuereinheit angesteuerten Antriebseinrichtung zur Vorschub- und/oder Krümmungssteuerung des Aktuators versehen. Die Basis-Steuereinheit kann also auf der Grundlage einer entsprechenden Regelungsschaltung oder nach Art eines CNC-Systems gestützt auf ein entsprechendes Steuerungsprogramm den Aktuator selbständig steuern und ihn bestimmte Punkte anfahren lassen. Als Eingangsgrößen für die Steuerung können dabei Informationen bezüglich eines Oberflächenkontaktes des distalen Endes an die Basis-Steuereinheit weitergegeben werden und direkt als Rückkopplungsgröße in die Steuerung einfließen. Dazu sind vorzugsweise am distalen Ende des Aktuators Berührungssensoren zur Erfassung eines mechanischen Kontaktes mit der Wand der Körperhöhlung angeordnet.

Ferner können in weiteren bevorzugten Ausführungsformen am distalen Ende des Aktuators Meßsensoren zur Erfassung medizinisch relevanter Meßwerte angeordnet werden. Diese Meßwerte können einerseits zu diagnostischen Zwecken dienen, andererseits können sie jedoch auch in die Positionssteuerung des Aktuators einfließen. So kann das Gerät beispielsweise im Herzen pathologische elektrische Signale durch Potentialmessung an der Herzwand orten. Durch ein Entlangtasten des Aktuators an der Herzwand und Messung der entsprechenden Potentialsignale kann das Gerät zu behandelnde Stellen ansteuern.

Denkbar ist auch die Plazierung automatisch im Bereich einer Stenose mittels Impedanzmessung im Gefäß zur Erfassung von Blutstrom und Gefäßvolumen.

Als alternative Datenbasis für die Steuerung des Aktuators können auch mit Hilfe von bildgebenden Verfahren aufgenommene dreidimensional kartographierte Koordinatenbilder der zu untersuchenden Körperhöhlung dienen, wobei die jeweiligen Ortskoordinaten des Aktuators in Beziehung gesetzt und entsprechende Steuerungsmaßnahmen gemäß dem "Fahrplan" des Aktuators ergriffen werden.

Schließlich ist es alternativ oder zusätzlich möglich, eine sogenannte "Navigationsmarke" als Referenzpunkt in oder in der Nähe der Körperhöhlung u.U. auch extrakorporal zu plazieren, die über entsprechende Sensoren des Aktuators erfaßbar ist. Auf dieser Basis können dann für die aktuelle Position des Aktuators relativ zur Navigationsmarke repräsentative Signale an die Basis-Steuereinheit abgegeben werden. Als eigentliche Antriebselemente für die Antriebseinrichtung des Aktuators kommen die bereits erwähnten Piezoelemente, aber auch elektrostriktive Polymer-Elemente (sogenannte "Nanotubes") genauso in Frage, wie pneumatische oder hydraulische Antriebselemente. Auch der Einsatz mikromechanischer Maschinenelemente in Nanotechnologie, also kleinste Zahnräder, Motoren, Getriebe u.s.w. ist möglich.

Weitere bevorzugte Ausführungsformen betreffen eine spezielle Problematik, die bei der Anwendung der erfindungsgemäßen Vorrichtung im oder am Herzen in prägnanter Weise auftritt. Das Herz als pumpendes Organ vollführt während des Schlagens starke Bewegungen. Herznahe Gefäße oder die Herzwände können also ohne weiteres relativ vehement mit einem in das Herz eingeführten Katheter kollidieren, was ein gewisses Verletzungsrisiko in sich birgt. Ferner kann die genaue Behandlungsposition durch das Schlagen nur sehr schwer oder ungenau eingehalten werden. Dies kann gerade bei sogenannten Ablationen - also dem Veröden von Herzmuskelgewebe zur Unterdrückung pathologischer Rhythmusstörungen - schwerwiegende Folgen haben.

Zur Vermeidung dieser Problematik kann nun die Nachführung des Aktuators an die mittels geeigneter Meß- oder Berührungssensoren detektierten Bewegungen der Körperhöhlung vorgesehen sein, wobei die Basis-Steuereinheit eine entsprechende Vorschub- und/oder Krümmungssteuerung des Aktuators vollzieht. Nähere Informationen hierzu sowie weitere Merkmale, Einzelheiten und Vorteile der Erfindung ergeben sich aus der nachfolgenden Beschreibung, in der Ausführungsbeispiele des Erfindungsgegenstandes näher erläutert werden. Es zeigen
- Fig. 1: ein schematisches Blockdiagramm der Gesamtvorrichtung,
- Fig. 2: ein Flußdiagramm zum Steuerungsablauf innerhalb der Steuereinheit,
- Fig. 3: ein schematisches Schaubild der Koordinatensteuerung der Vorrichtung,
- Fig. 4 und 5: Seitenansichten des distalen Endes eines Aktuators in zwei unterschiedlichen Stellungen,
- Fig. 6 und 7: analoge Ansichten eines Aktuators in einer zweiten Ausführungsform,
- Fig. 8 und 9: schematische Schnittdarstellungen eines piezeoelektrischen Stick-and-Slip-Vorschubelementes,
- Fig. 10: ein schematisches Schaubild des Herzens mit eingeführtem, der Herzbewegung nachführbaren Aktuator, und
- Fig. 11: ein Flußdiagramm zum Steuerungsablauf innerhalb der Steuereinheit bei der Nachführung des Aktuators.

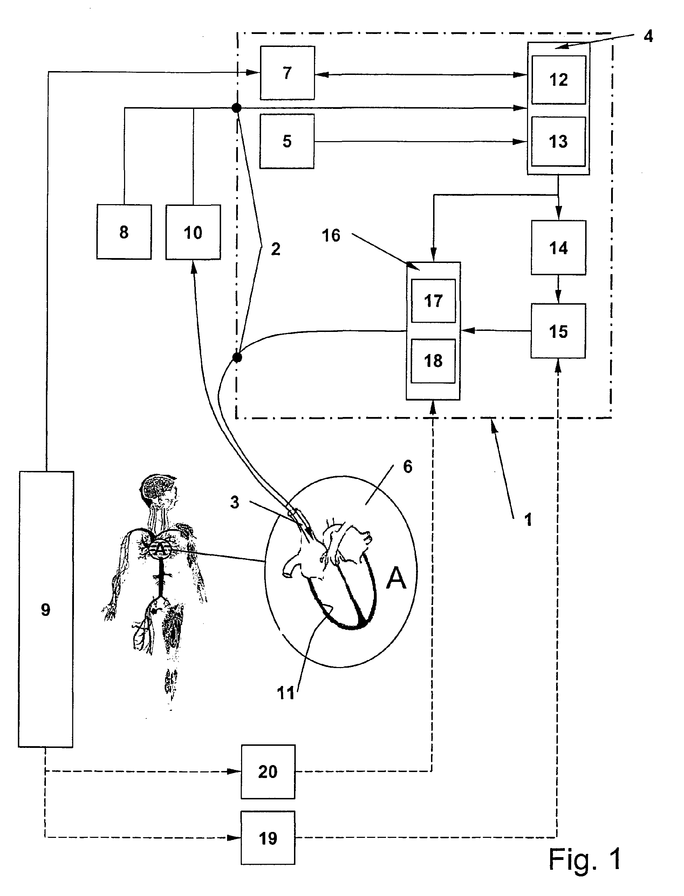

Anhand von Fig. 1 ist das Gesamtkonzept einer erfindungsgemäßen Vorrichtung zur automatischen Durchführung von medizinischen Aktionen in Körperhöhlungen zu erläutern. So ist grundsätzlich eine als Ganzes mit 1 bezeichnete Basis-Steuereinheit vorgesehen, die die innerhalb der strichpunktierten Umrandung zusammengefaßten Komponenten aufweist. Diese Basis-Steuereinheit 1 ist beispielsweise eine PC-gestützte Steuereinheit mit einer entsprechenden Schnittstelle 2 zu einem mit ihr gekoppelten Aktuator 3, der im vorliegenden Ausführungsbeispiel als Herzkatheter ausgebildet ist. Er repräsentiert jedoch unterschiedlichste Arten von Aktuatoren, wie Endoskope, Elektroden oder Kanülen.

Die Basis-Steuereinheit 1 ist in verschiedene Funktionsbereiche zu unterteilen. Es sind dies einerseits der Positionserfassungsblock 4, der im wesentlichen für die Erfassung der aktuellen Position des Aktuators 3 auf der Basis verschiedenster Eingangsgrößen zuständig ist. Dazu gehören einerseits eine Vermessungseinrichtung 5, mit deren Hilfe eine entsprechende Körperregion, wie das Herz 6, dreidimensional mittels Ultraschall oder Röntgenstrahlung erfaßt und entsprechende Koordinatendaten der Herzstrukturen ermittelt werden. Die entsprechenden Daten sind in einem Speicher 7 abgelegt.

In einer modifizierten Ausführungsform kann auch die betreffende Körperhöhlung zunächst mittels eines bildgebenden Verfahrens (z.B. Röntgen oder Ultraschall) einerseits in ihrer Lage und Form bestimmt werden und andererseits die durch die Patientenbewegungen (Herzschlag, Atmung) hervorgerufenen Änderungen der Lage und Form der Höhlung ausgemessen werden. Da diese Bewegungen periodischer Natur sind, ist es in bestimmten Fällen möglich, Lage und Form der zu untersuchenden Körperhöhlung durch die Steuereinheit zu jedem Zeitpunkt im Voraus zu berechnen, ohne dabei den Patienten erneut durch das bildgebende Verfahren zu belasten.

Ferner ist dem Positionserfassungsblock 4 eine Navigationseinrichtung in Form z.B. einer Navigationsmarke 8 zugeordnet, die in Fig. 1 zwar als Diagrammblock dargestellt, jedoch tatsächlich beispielsweise im Koronarsinus des Herzens 6 oder extrakorporal vom Operateur 9 plaziert ist. Über die erwähnte Schnittstelle 2 ist die Navigationsmarke angebunden. Über entsprechende, hier nicht näher dargestellte Sensoren am Aktuator 3 können die relativen Koordinaten zwischen Aktuatorspitze und Navigationsmarke 8 festgestellt und dem Positionserfassungsblock 4 übermittelt werden.

Schließlich ist am Aktuator 3 ein Berührungssensor 10 (ebenfalls wieder als Diagrammblock dargestellt) vorgesehen, der einen mechanischen Kontakt des Aktuators 3 mit beispielsweise der Außenwand 11 des rechten Ventrikels detektiert und ein entsprechendes Signal über die Schnittstelle 2 an den Positionserfassungsblock 4 gibt.

Statt eines Berührungssensors 10 kann auch ein Wandabstandssensor eingesetzt werden, der die Entfernung zwischen Aktuator 3 und der Wand des Herzens detektiert. Dies ist beispielsweise dann interessant, wenn der Aktuator einen Dilatationsballon trägt. Ein anderer Anwendungsfall ist die Brachytherapie, bei der der Aktuator 3 mittig im Gefäß sitzen muß.

Der Positionserfassungsblock 4 weist entsprechend der Anbindung von Vermessungseinrichtung 5, Navigationseinrichtung 8 und Berührungssensor 10 eine Orientierungseinheit 12 und eine Berührungserfassungseinheit 13 auf. Die Orientierungseinheit 12 verarbeitet die Koordinatendaten der Vermessungseinrichtung 5 bzw. der entsprechenden Daten aus dem Speicher 7 und setzt sie zu den von der Navigationseinrichtung 8 bereitgestellten Daten in Beziehung. Entsprechend verarbeitet die Berührungserfassungseinheit 13 die vom Berührungssensor 10 gelieferten Daten und bereitet sie auf.

Zur Verarbeitung der Daten von Orienterierungs- 12 und Berührungserfassungseinheit 13 dient eine zentrale Steuereinheit 14, die aufgrund eines Steuerprogrammes eine entsprechende Regelung 15 für die Vortriebsparameter, nämlich Vorschublänge und Richtung des Aktuators 3 berechnet.

Mit diesen Daten wird nun der Ausgangsblock 16 angesteuert, der einen Vorschubtreiber 17 zur Ansteuerung der entsprechenden Antriebselemente im Aktuator 3 zu dessen Vorschub und Krümmungsveränderung aufweist. Ferner ist ein Treiber 18 für die im Aktuator 3 vorhandenen Behandlungsfunktionen, wie z.B. eine Ablationselektrode oder ein Dilatationsballon vorgesehen.

Falls der Operateur 9 manuell in den Geräteablauf eingreifen will, sind Eingabeeinheiten 19, 20 zum Zugriff auf die Regelung bzw. den Ausgabeblock 16 vorgesehen.

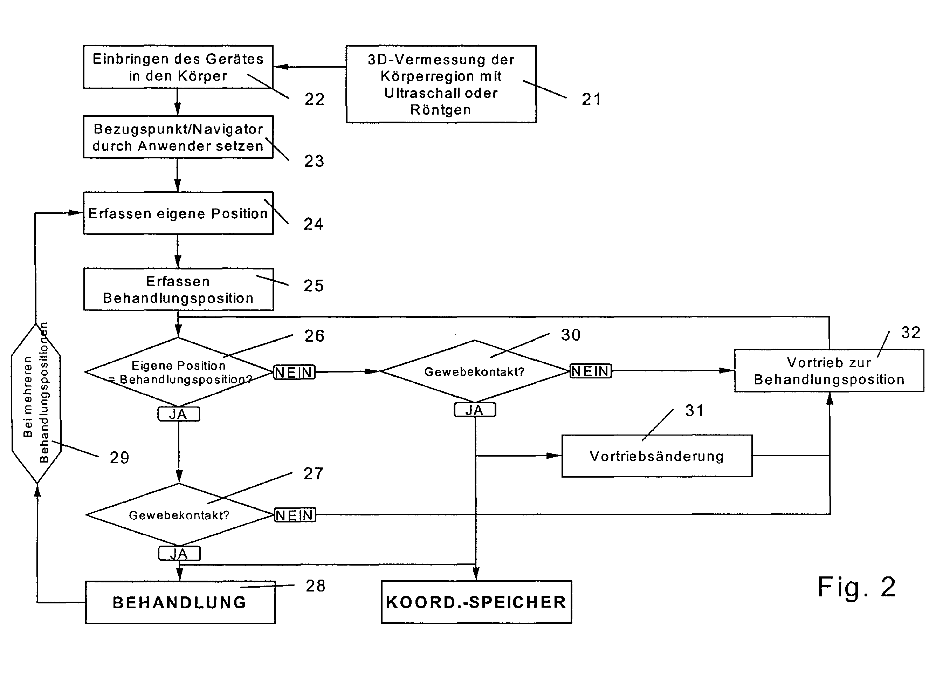

Anhand von Fig. 2 ist der praktische Einsatz der erfindungsgemäßen Diagnose- und Therapie-Vorrichtung anhand eines Flußdiagrammes zu erläutern. Nach einer dreidimensionalen Vermessung der zu untersuchenden Körperregion mit Ultraschall oder Röntgenstrahlung (Block 21) wird der Aktuator in den Körper eingesetzt (Block 22). Dies erfolgt beispielsweise durch Einschieben eines den Aktuator repräsentierenden Katheters in das rechte Atrium des Herzens. Anschließend wird beispielsweise im Koronarsinus durch den Operateur 9 die Navigationsmarke 8 gesetzt (Block 23). Durch ein Inbeziehungsetzen des distalen Endes des in das Herz eingeführten Katheters zu dieser Navigationsmarke 8 wird die eigene Position des distalen Endes des Katheters mit Hilfe des Positionserfassungsblocks 4 erfaßt (Block 24). Durch eine entsprechende Eingabe wird die eigentliche Behandlungsposition festgelegt und in der Steuereinheit 14 definiert. Dadurch läßt sich als nächster Schritt die Behandlungsposition erfassen (Block 25).

Bei der anschließenden Abfrage wird im Programmablauf festgestellt, ob die Position des Aktuators der Behandlungsposition entspricht. Falls ja, wird eine Abfrage 27 durchgeführt, ob das distale Ende des Aktuators 3 Kontakt mit Gewebe hat. Falls ja, kann die eigentliche Behandlung (Block 28) erfolgen, indem beispielsweise eine Ablationselektrode zur Verödung oder zum Entfernen von Herzgewebe aktiviert wird. Sollen mehrere Behandlungen an verschiedenen Positionen durchgeführt werden, so wird der Prozeß über die Rückkopplung 29 zu Schritt 24 zurückgeführt.

Falls bei der Abfrage 26 festgestellt wurde, daß die Behandlungsposition noch nicht erreicht ist, wird wiederum über eine Abfrage 30 ermittelt, ob der Aktuator Gewebekontakt hat. Falls ja, wird über den Zugriff auf den Koordinatenspeicher 7 eine Änderung des Vortriebes (Block 31) vorgenommen, um den Aktuator entsprechend in Richtung der Soll-Behandlungsposition vorzuschieben (Block 32). Diese Abfrageschleife ausgehend von der Abfrage 26 wird so lange durchgeführt, bis bei der Abfrage 26 festgestellt wird, daß die Position des Aktuators mit der Behandlungsposition übereinstimmt. In diesem Zusammenhang ist zu erwähnen, daß die Vermessung des Herzens 6 nicht nur über die vorstehend erwähnten bildgebenden Verfahren, wie Ultraschall oder Röntgenstrahlung möglich ist. Alternativ oder zusätzlich kann die Vermessung des Herzens auch mit Hilfe des Berührungssensors 10 erfolgen. Sobald dieser nämlich einen Kontakt zur Wand des Herzens feststellt, können die entsprechenden Koordinaten in den Koordinatenspeicher 7 eingelesen werden, so daß sich durch entsprechendes Abtasten der Herzwand mit dem Aktuator 3 ein topologisches Bild der die jeweilige Körperhöhlung umgrenzenden Wände ergibt.

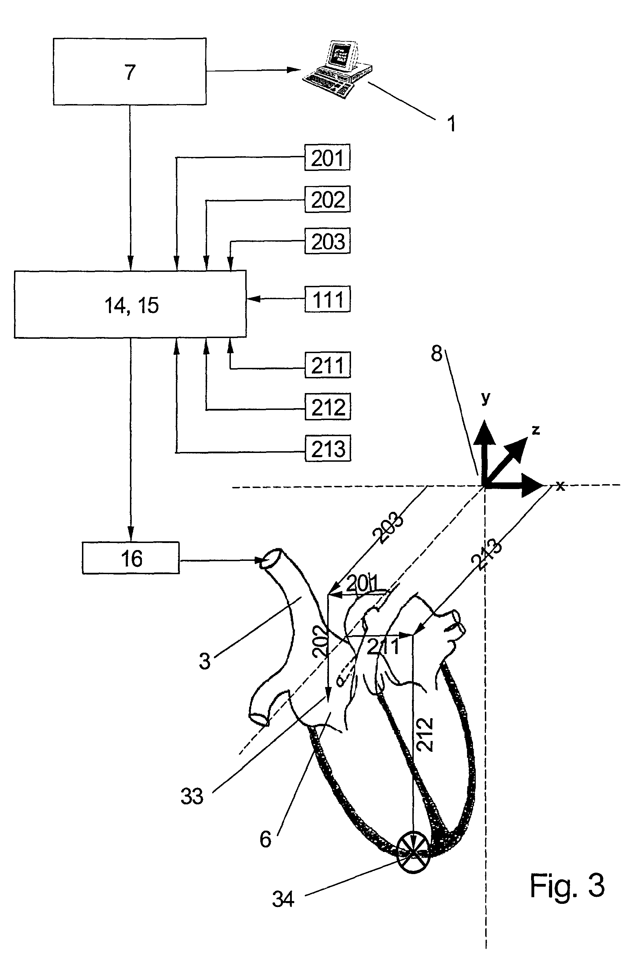

Anhand von Fig. 3 ist die Navigationsroutine beim Positionieren des Aktuators 3 innerhalb des Herzens zu erläutern. So ist der Aktuator 3 im Bereich seines distalen Endes mit einem Lagebestimmungselement 33 versehen, mit dessen Hilfe die Positionskoordinaten 201, 202, 203 des Aktuators 3 im Verhältnis zu der Navigationseinrichtung 8, die in Fig. 3 durch das Koordinatenkreuz x-y-z repräsentiert ist, bestimmt werden. Die durch ein Kreuz gekennzeichnete Behandlungsposition 34 weist die Koordinaten 211, 212, 213 auf. Aus einem Vergleich der Koordinaten 201, 202, 203 einerseits und 211, 212, 213 andererseits werden von der Steuerung 14 bzw. Regelung 15 die Richtung und der Betrag des notwendigen Vorschubes des Aktuators 3 bestimmt. Dabei fließt gleichzeitig ein Berührungssignal 111 ein, das aktiv ist, wenn das distale Ende des Aktuators 3 an eine Wand des Herzens 6 anstößt. Schließlich wird zur Bestimmung der Vorschubkoordinaten die im Speicher 7 der Basis-Steuereinheit 1 abgelegten Vermessungskoordinaten des Herzens mit einbezogen, damit ein Anstoßen des distalen Endes auf den Weg zur Behandlungsposition 34 möglichst von vornherein vermieden wird.

Die entsprechend ermittelten Steuerungsdaten für den Aktuator 3 werden dem Ausgangsblock 16 zugeführt, der die physische Steuerung des Aktuators durch Aktivierung entsprechender Vorschubelemente bewerkstelligt.

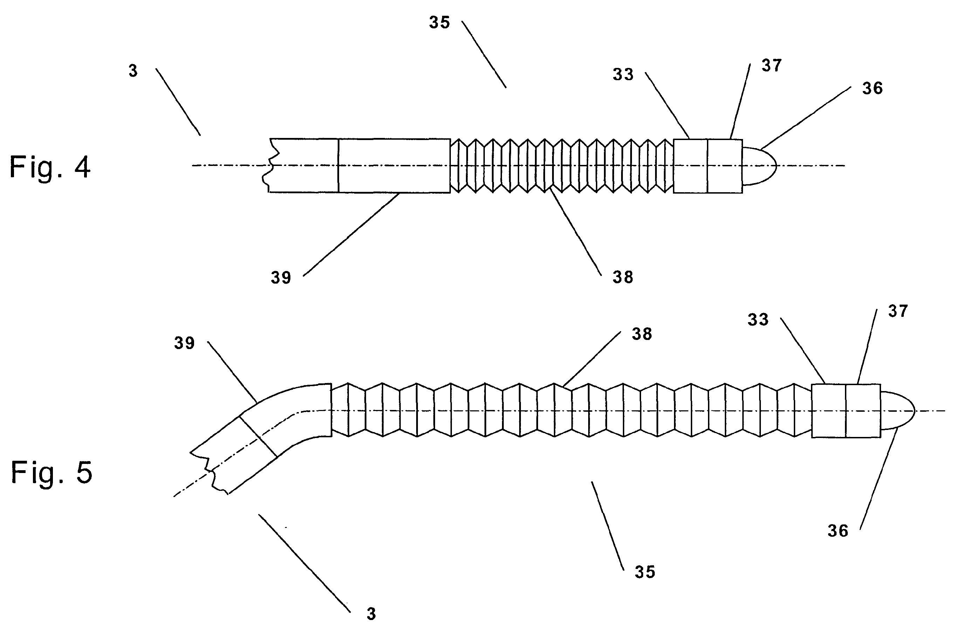

In den Fig. 4 und 5 ist das distale Ende 35 eines Aktuators 3 gezeigt. So ist direkt am Ende eine Ablationselektrode 36 angedeutet, mit deren Hilfe eine entsprechende therapeutische Behandlung im Herzen vorgenommen werden kann. Es schließt sich ein Berührungssensor 37 in Form einer Ringelektrode an. Der darauffolgende Ring stellt das Lagebestimmungselement 33 des Aktuators dar. Es schließt sich ein längenveränderliches Element 38 an, das beispielsweise durch einen später noch beschriebenen piezoelektrischen Antrieb ausschiebar und einziehbar ist. In Fig. 5 ist die gegenüber Fig. 4 ausgefahrene Stellung des längenveränderlichen Elementes 38 gezeigt und durch die auseinandergezogenen Balgenfalten verdeutlicht.

An das längenveränderliche Element 38 schließt sich ein biegbares Element 39 an, das in seinem Inneren ebenfalls über den Umfang verteilte Piezoelemente aufweist. Durch selektives Ansteuern einzelner Piezoelemente kann das biegbare Element aus dem gestreckten Zustand in Fig. 4 in einen gebogenen Zustand (Fig. 5) übergeführt werden.

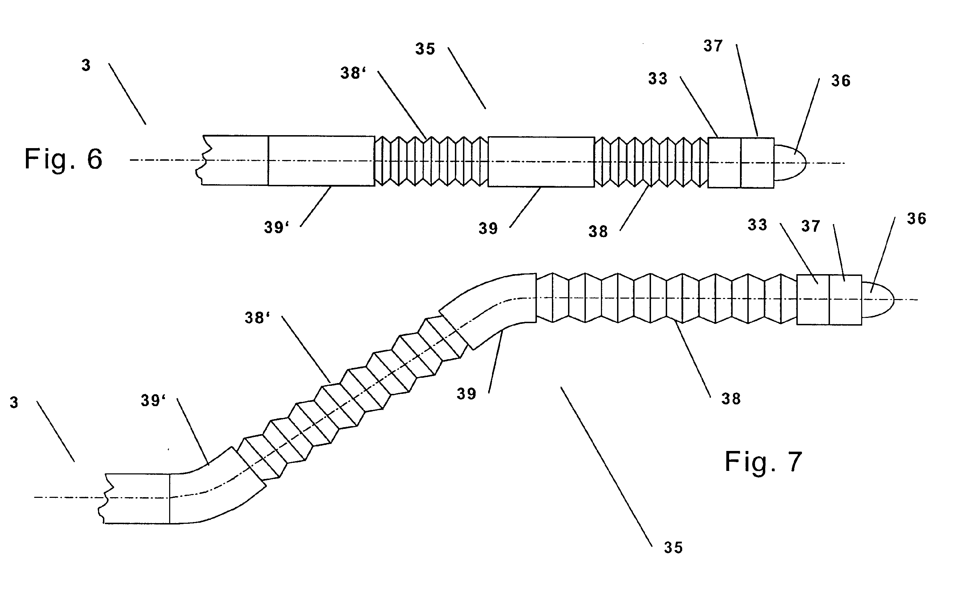

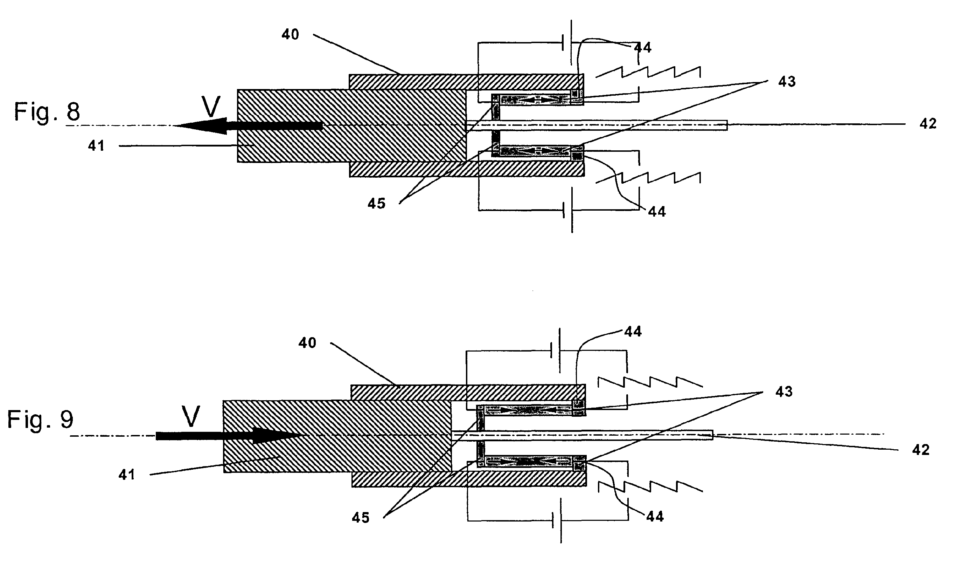

Die in Fig. 6 und 7 gezeigte Version unterscheidet sich von der gemäß Fig. 4 und 5 lediglich darin, daß zwei längenveränderliche Elemente 38, 38' und zwei biegbare Elemente 39, 39' vorgesehen sind. Im übrigen kann auf die Beschreibung zu Fig. 4 und 5 verwiesen werden.

Im übrigen ist darauf hinzuweisen, daß die erwähnten Piezoelemente oder elektrostriktiven Elemente zur Längen- und Krümmungsänderung mit mechanischen Zugdrähten kombiniert werden können. So können auf der einen Seite des Aktuators 3 durch Anlegen einer Spannung an die erwähnten Elemente die Außenwandbereiche versteift werden, während auf der anderen Seite des Aktuators 3 die elastisch gebliebene Wand mit einem Zugdraht eingeknickt wird. Ist das distale Ende um seine eigene Achse drehbar, kann dann der Aktuator in praktisch jede beliebige Raumrichtung vorwärts geschoben werden. Um die Drehbarkeit zu vermeiden, können auch mehrere Piezo- oder elektrostriktive Elemente und Zugdrähte eingesetzt werden.

In den Fig. 8 bis 10 ist ein mikromechanisches piezoelektrisches Antriebselement in Form eines sogenannten Stick-and-Slip-Antriebselementes dargestellt. So ist in einer äußeren Lagerhülse 40 ein Schieber 41 längsverschiebbar gelagert, der beispielsweise mit dem distalen Ende des Aktuators 3 in Verbindung steht. Am inneren Ende des Schiebers 41 ist ein Schubelement 42 in Längsrichtung des Schiebers 41 angebracht. Um das Schubelement 42 herum sind Piezoelemente 43 positioniert, die mit ihrem rückwärtigen Ende über ein Widerlager 44 fest mit der Lagerhülse 40 verbunden sind. Das freie Ende der Piezoelemente 43 ist mit einem Kontaktvorsprung 45, z.B. aus einer Art Neopren-Material versehen, das in Reibschluß mit dem Schubelement 42 steht. Werden nun die Piezoelemente 43 mit einer sägezahnförmigen Spannung mit flach ansteigenden und steil abfallenden Flanken beaufschlagt (Fig. 8), so erfolgt die Längenausdehnung der Piezoelemente 43 langsam und die Längenkontraktion schnell. Durch die langsame Längenausdehnung wird das Schubelement 42 mitgenommen ("Stick") in Vorschubrichtung V gemäß Fig. 8 vorgeschoben, kann dabei jedoch der raschen Kontraktion in Gegenrichtung ("Slip") nicht folgen, so daß sich netto eine Verschiebung des Schubelementes 42 und damit des Schiebers 41 in Vorschubrichtung V ergibt.

Wird die Sägezahnspannung umgekehrt, d.h. mit steil ansteigenden und flach abfallenden Flanken aufgebracht, so nehmen umgekehrt die Kontaktvorsprünge 45 das Schubelement 42 in Vorschubrichtung V' gemäß Fig. 9 mit, wogegen beim schnellen Ausdehnen das Schubelement 42 wiederum nicht folgen kann. Netto ergibt sich eine Bewegung in Vorschubrichtung V' gemäß Fig. 9 des Schiebers 41.

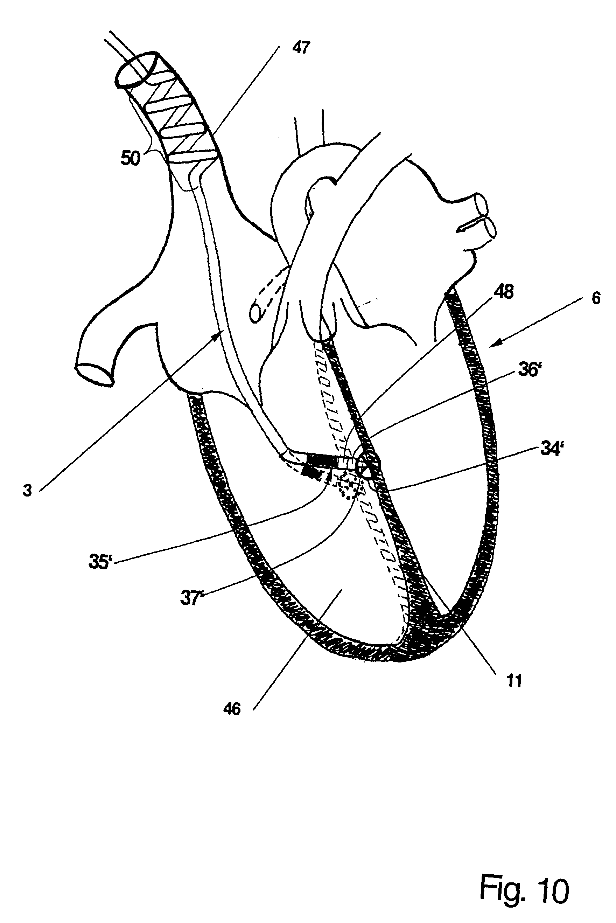

In Fig. 10 ist ein als Ablator ausgebildeter Aktuator 3' in seiner in das rechte Ventrikel 46 des Herzens 6 eingeführten Position gezeigt. Das distale Ende 35' weist dabei neben der eigentlichen Ablationselektrode 36' einen Meßsensor 48, z.B. in Form eines Ultraschallsensors auf, der den Abstand des distalen Endes 35' des Aktuators 3' von der Herzscheidewand 11' mißt. Dadurch wird die Bewegung der Herzscheidewand 11' in Amplitude und Frequenz detektiert und entsprechende Daten der Basis-Steuereinheit 1 (nicht gezeigt in Fig. 10) übermittelt. Diese berechnet daraus entsprechende Steuerungsdaten für das distale Ende 35' des Aktuators 3', das - wie vorstehend ausführlich anhand des Aktuators 3 erläutert - mit entsprechenden Antriebseinrichtungen zur Vorschub- und Krümmungssteuerung des Aktuators versehen ist. Entsprechend kann der Aktuator 3' gleichförmig zum sich bewegenden Herzmuskelgewebe bzw. zur gewünschten Behandlungsstelle bewegt werden, ohne daß es zu unerwünschten Kollisionen mit dem Myokardgewebe des Herzens kommt.

Um die Genauigkeit dieses Vorgangs zu gewährleisten, weist der Aktuator 3' einen spiralförmig aufweitbaren medialen Abschnitt 50 auf. Derartige Katheterabschnitte sind an sich bekannt und dienen zur Verankerung des Aktuators 3' beispielsweise in der Vena cava 47, wie dies in Fig. 10 gezeigt ist.

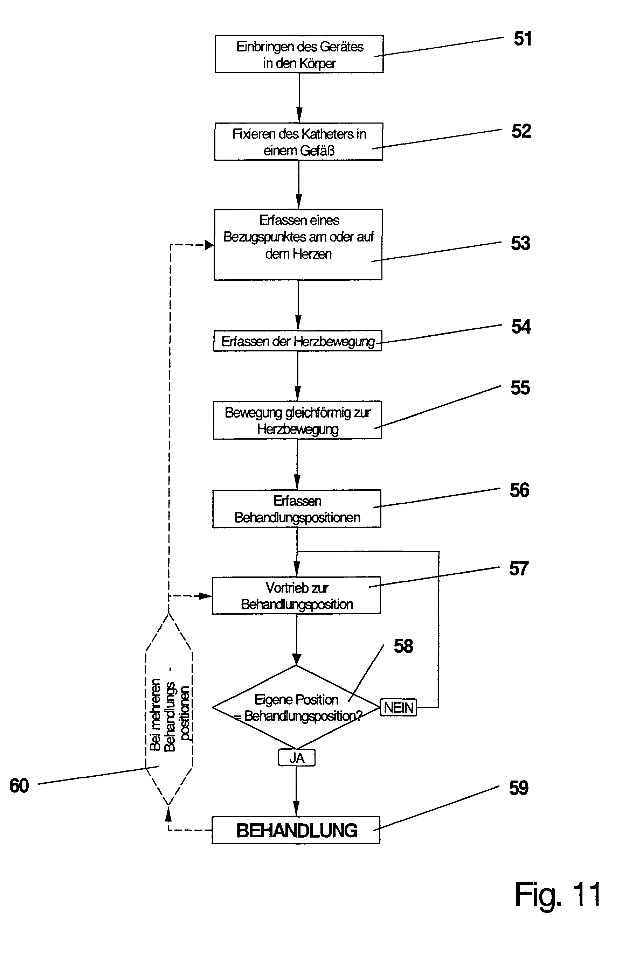

Anhand von Fig. 11 ist nun das Vorgehen während des Einbringens und Einsetzens des Aktuators 3' in das gezeigte Herz zu erläutern.

So wird in einem Schritt 51 der katheterförmige Aktuator 3' über eine Vene bis in das Herz 6 vorgeschoben. Dabei ist in den Katheter ein Mandrin-Draht eingeführt, so daß der spiralförmige mediale Abschnitt 50 gestreckt ist. Ist der Aktuator 3' in seiner Grundposition im Herzen 6 angelangt, wird der Mandrin-Draht aus dem Aktuator 3' entfernt und dieser somit durch das damit verbundene Aufweiten des spiralförmigen medialen Abschnittes 50 in dem entsprechenden Gefäß 47 fixiert (Schritt 52).

Statt der üblichen Verwendung eines Mandrin-Drahtes zur Steuerung der Deformation des medialen Abschnittes 50 können dort auch in analoger Weise wie im Bereich seines distalen Endes 35' Antriebseinrichtungen im Form von Piezoelementen, elektrostriktiven Polymer-Elementen, mikromechanischen Antriebselemente oder dergleichen eingebaut sein, die von der Basis-Steuereinheit automatisch ansteuerbar und damit aktivierbar sind. Durch die entsprechend erzeugbare Deformation des medialen Abschnittes 50 wird der katheterförmige Aktuator 3' im Gefäß 47 fixiert.

Nach diesem Einführen und Fixieren des Aktuators 3' ragt dessen distales Ende 35' frei in das Herz 6 hinein. Ab diesem Zeitpunkt kann der Aktuator 3' dann im wesentlichen selbständig und ohne Hilfe eines Operateurs arbeiten. Der Aktuator sucht sich gesteuert von der Basis-Steuereinheit 1 einen Bezugspunkt am Myokard (Schritt 53) und erfaßt danach die Herzbewegung über den Meßsensor 48 (Schritt 54). Anschließend wird der Aktuator 3' über die entsprechenden Antriebseinrichtungen in seinem distalen Ende von der Basis-Steuereinheit 1 gleichförmig zur ermittelten Herzbewegung gesteuert, was strichliert in Fig. 10 angedeutet ist. Unter Beibehaltung dieses Schrittes 55 werden anschließend die eine oder mehrere Behandlungspositionen erfaßt (Schritt 56), indem über geeignete Sensoren beispielsweise pathologische elektrische Signale gesucht werden. Auf diese Weise kann ein Ablationskatheter notwendige Verödungsstellen finden. Weitere Beispiele für Steuerparameter zum Auffinden von Behandlungspositionen sind die Strömungsgeschwindigkeit beispielsweise in den Herzkranzgefäßen, wenn ein Ballonkatheter zum Ermitteln der zu dilatierenden Stelle gesteuert werden soll.

Anschließend erfolgt der Vortrieb des Aktuators 3' zur gewünschten Behandlungsposition 34' (Schritt 57). Dabei erfolgt regelmäßig eine Abfrage 58, ob die eigene Position der Behandlungsposition entspricht. Diese Abfrage findet über die Navigationseinrichtung und Orientierungseinheit des Gerätes statt, wie er anhand der Fig. 1 näher erörtert wurde. Falls die eigene Position nicht der Behandlungsposition entspricht, so läuft der Prozeß zyklisch zum Schritt 57 zurück.

Ist die Behandlungsposition erreicht, so erfolgt die eigentliche Behandlung gemäß Schritt 59. Falls mehrere Behandlungspositionen angesteuert werden sollen, wird der Prozeß über die Rückkopplung 60 entweder zu Schritt 57 oder Schritt 53 zurückgekoppelt, wie kurz bzw. lang gestrichelt in Fig. 11 angedeutet ist.

## Patentansprüche

1. Vorrichtung zur automatischen Durchführung von diagnostischen und/oder therapeutischen Aktionen in Körperhöhlungen (6) mit
- einer Basis-Steuereinheit (1), und
- einem damit gekoppelten, in die entsprechende Körperhöhlung einführbaren, katheterartigen Aktuator (3 3'), der
= im Bereich seines distalen Endes (35, 35') mit einer diagnostisch aktiven Detektor- und/oder therapeutisch aktiven Behandlungseinrichtung (36, 47') versehen ist, sowie
= zumindest im Bereich seines distalen Endes (35, 35') mit einer von der Basis-Steuereinheit (1) angesteuerten Antriebseinrichtung (43) zur Vorschub- und/oder Krümmungssteuerung des Aktuators (3, 3') versehen ist.

2. Vorrichtung nach Anspruch 1, **dadurch gekennzeichnet, daß** der Aktuator (3) an seinem distalen Ende (35) mit einem Meßsensor, insbesondere einer Meßelektrode zur Erfassung medizinisch relevanter Meßwerte, als diagnostisch aktivem Detektor versehen ist.

3. Vorrichtung nach Anspruch 1 oder 2, **dadurch gekennzeichnet, daß** der Aktuator (3, 3') an seinem distalen Ende mit einer Ablationselektrode (36, 36'), einem Dilatationsballon oder dergleichen als therapeutisch aktiver Behandlungseinrichtung versehen ist.

4. Vorrichtung nach einem der Ansprüche 1 bis 3, **dadurch gekennzeichnet, daß** der Aktuator (3, 3') mit einem Berührungssensor (10, 37, 37') zur Erfassung eines mechanischen Kontaktes mit der Wand (11, 49) der Körperhöhlung (6) versehen ist.

5. Vorrichtung nach einem der Ansprüche 1 bis 4, **dadurch gekennzeichnet, daß** die Antriebseinrichtung für den Aktuator (3, 3') durch elektromechanisch arbeitende Vorschubelemente (43), insbesondere durch Piezoelemente, elektrostriktive Polymer-Elemente, mikromechanische Antriebselemente oder dergleichen gebildet ist.

6. Vorrichtung nach einem der Ansprüche 1 bis 5, **dadurch gekennzeichnet, daß** die Antriebseinrichtung für den Aktuator (3, 3') durch pneumatische oder hydraulische Antriebselemente gebildet ist.

7. Vorrichtung nach einem der Ansprüche 1 bis 6, **dadurch gekennzeichnet, daß** die Basis-Steuereinheit (1) eine Orientierungseinheit (12) zur Vorschub- und Richtungssteuerung des Aktuators (3, 3') aufweist, wobei in einem Speicher (7) ein durch Vermessung ermitteltes Koordinatenbild der entsprechenden Körperhöhlung (6) als Basis für die Aktuator-Steuerung abgelegt ist.

8. Vorrichtung nach Anspruch 7, **dadurch gekennzeichnet, daß** die im Speicher (7) abgelegten Koordinaten durch Vermessung mit Hilfe bildgebender Verfahren, insbesondere durch Ultraschall oder Röntgenstrahlung, ermittelt sind.

9. Vorrichtung nach Anspruch 7 oder 8, **dadurch gekennzeichnet, daß** die im Speicher (7) abgelegten Koordinaten durch eine Vermessung mit Hilfe des Berührungssensors (10, 37, 37') ermittelt sind.

10. Vorrichtung nach Anspruch 9, **dadurch gekennzeichnet, daß** mit Hilfe des Berührungssensors (10, 37) Bewegungen der zu vermessenden Körperhöhlung (6) ermittelbar und in die Vermessung einbeziehbar sind.

11. Vorrichtung nach einem der Ansprüche 1 bis 10, **dadurch gekennzeichnet, daß** eine in oder in der Nähe der Körperhöhlung (6) plazierbare Referenz-Navigationsmarke (8) vorgesehen ist, die als relativer Bezugspunkt für die Positionsbestimmung des Aktuators (3) fungiert.

12. Vorrichtung nach einem der Ansprüche 1 bis 11, **dadurch gekennzeichnet, daß** die Antriebseinrichtung für den Aktuator (3, 3') von piezoelektrischen Stick-and-slip-Antriebselementen (42, 43, 44, 45) gebildet ist.

13. Vorrichtung nach einem der Ansprüche 1 bis 12, **dadurch gekennzeichnet, daß** mittels eines Meß- oder Berührungssensors (48) die Bewegungen der Körperhöhlung (6) detektierbar und von der Basis-Steuereinheit (1) derart in die Vorschub- und/oder Krümmungssteuerung des Aktuators (3)einbeziehbar sind, daß das distale Ende (35') des Aktuators (3') den Bewegungen der Körperhöhlung (6) nachführbar ist.

14. Vorrichtung nach einem der Ansprüche 1 bis 13, **dadurch gekennzeichnet, daß** der Aktuator (3') einen aufweitbaren medialen Abschnitt (50) zu seiner Verankerung in einer Körperhöhlung (6) aufweist.

15. Vorrichtung nach Anspruch 14, **dadurch gekennzeichnet, daß** der mediale Abschnitt (50) spiral- oder wellenförmig zur Verankerung in einem Blutgefäß (47) aufweitbar ist.

16. Vorrichtung nach Anspruch 14 oder 15, **dadurch gekennzeichnet, daß** die Deformation des medialen Abschnitts (50) des Aktuators (3') durch automatisch ansteuerbare Antriebseinrichtungen, insbesondere durch Piezoelemente, elektrostriktive Polymer-Elemente, mikromechanische Antriebselemente oder dergleichen, erzeugbar ist.
